# EUROPÄISCHE PATENTSCHRIFT

(11) **EP 2 861 169 B1**
(45) Veröffentlichungstag und Bekanntmachung des Hinweises auf die Patenterteilung: **15.11.2017**
(21) Anmeldenummer: 13711831.1
(22) Anmeldetag: 13.03.2013
(51) Int. Cl.: A61B 17/68, A61B 17/80

(54) **FIXIERUNGSVORRICHTUNG ZUM FIXIEREN VON BRUCHENDEN VON KNOCHEN EINER KNOCHENFRAKTUR**
FIXING DEVICE FOR FIXING THE FRACTURE ENDS OF THE BONES IN A BONE FRACTURE
DISPOSITIF DE FIXATION POUR FIXER DES EXTRÉMITÉS D'OS FRACTURÉES D'UNE FRACTURE OSSEUSE

(30) Priorität: 13.06.2012 DE 102012105123
(43) Veröffentlichungstag der Anmeldung: 22.04.2015
(73) Patentinhaber: Hipp Medical AG, 78600 Kolbingen (DE)
(72) Erfinder: WAIZENEGGER, Markus, 78600 Kolbingen (DE)
(74) Vertreter: Kuhnen & Wacker
(86) Internationale Anmeldenummer: PCT/EP2013/000750
(87) Internationale Veröffentlichungsnummer: WO 2013/185864

(56) Entgegenhaltungen:
- DE-A1-102007 010 950
- DE-B3-102006 020 905
- DE-U1-202011 109 808
- US-A- 5 275 601
- US-A1- 2004 073 215
- US-A1- 2007 250 171
- US-A1- 2007 270 820
- US-A1- 2009 036 930
- US-A1- 2011 251 691
- US-B1- 6 432 017

## Beschreibung

Die Erfindung betrifft eine Fixierungsvorrichtung zum Fixieren von Bruchenden von Knochen einer Knochenfraktur nach Anspruch 1. Bei unzureichender Ruhigstellung einer Knochenfraktur kann eine Kallusbildung, d.h. eine schwielenartige Verdickung der Bruchenden aus überwachsendem Knochengewebe, auftreten. Um eine solche indirekte Frakturheilung über einen Kallus zu vermeiden, werden Knochenplatten verwendet, die auf der Außenfläche eines gebrochenen Knochens aufgebracht und befestigt werden, damit die Bruchstelle während des Heilungsprozesses fixiert ist.

Zudem wird der Heilungsprozess einer Fraktur günstig beeinflusst, wenn eine Kompression auf die zusammengefügte Bruchstelle aufgebracht wird. Hierdurch wird eine besonders enge Adaption, d.h. ein geringes Spaltmaß, über welches die Bruchenden wieder aufeinander zu wachsen, bewirkt.

Die US 2009/0036930 A1 offenbart eine Knochenfixiervorrichtung, mit der Bruchenden von Knochen einer Knochenfraktur während des Heilungsprozesses unter Kompression fixiert werden können. Durch Aneinanderreihen von mehreren Knochenfixiervorrichtungen kann ein modulares Fixiersystem ausgebildet werden. Allerdings können die mehreren das modulare Fixiersystem ausbildenden Knochenfixiervorrichtungen nicht vor dem Einbringen der Befestigungsmittel in die jeweiligen Bruchenden in einer vorbestimmten Lage zueinander positioniert werden. Eine zueinander ausgerichtete Positionierung der Knochenfixiervorrichtungen ist hier erst nach Einbringen der Befestigungsmittel in die jeweiligen Bruchenden der Knochen möglich.

Ferner ist aus der DE 20 2011 109808 U1 ein Spannelement bekannt, das aus einem durch eine umlaufende Wandung ausgebildeten Konturkörper mit federelastischen Beugeabschnitten und Aufnahmeabschnitten besteht. Dabei können auch mehrere Spannelemente mittels Durchbrüche und Ausschnitte in deren Aufnahmeabschnitten im Sinne einer Nut- und Feder-Verbindung verbunden werden. Durch das Verbinden mehrerer Spannelemente ist eine modulare Konfiguration vorgesehen, anhand der eine Fixierungsvorrichtung an die Struktur eines Bruchs sowie an die lokalen anatomischen Begebenheiten individuell angepasst werden kann. Nachteilig stellt sich bei dem hier diskutierten Spannelement ebenfalls, wie bei dem vorstehenden Stand der Technik, heraus, dass die verbundenen Spannelemente vor dem Hindurchstecken der Befestigungsmittel durch die sich benachbarten Spannelemente und dem anschließenden Einbringen der Befestigungsmittel in die Bruchenden nicht im Voraus fest zueinander positioniert werden können. Die Anpassung an die Struktur eines Bruchs sowie an die lokalen anatomischen Begebenheiten kann während einer Operation erst beim direkten Fixieren der Spannelemente an den Knochen geschehen.

Dementsprechend ist es Aufgabe der vorliegenden Erfindung, eine Fixierungsvorrichtung zu schaffen, mit der die feste Ausrichtung mehrer Spannelemente zueinander bereits vor dem Fixieren der einzelnen Spannelemente an den Bruchenden der Knochen möglich ist.

Diese Aufgabe wird durch eine Fixierungsvorrichtung mit den Merkmalen des Anspruchs 1 gelöst.

So können die Spannelemente bereits im Voraus, z.B. anhand der aus vorausgegangenen Untersuchungen gewonnenen Daten oder Erkenntnissen besonders gut vor dem Einbringen der Befestigungsmittel in die Bruchenden zueinander unter einem vorbestimmten Winkel ausgerichtet werden.

Die Anpassung der Spannelemente an die Struktur eines Bruchs sowie an die lokalen anatomischen Begebenheiten kann somit vor dem direkten Fixieren der Spannelemente an den Knochen geschehen. Mittels eines Bildgebungsverfahrens, wie z.B. durch herkömmliche Röntgentomographie, Computertomographie (CT), Magnetresonanztomographie (MRT), Positronen-Emissions-Tomographie (PET) oder dergleichen, kann somit die Struktur der Fraktur analysiert werden und vorab die die Bruchenden der Knochen zusammenhaltenden Spannelemente unter der gewünschten Winkellage ausgerichtet werden. Es kann demzufolge eine vordefinierte Fixierungsvorrichtung als Spannelemente-Modularsystem konfiguriert werden.

Gerade bei komplexen Frakturen, welche eine aufwendige Vorbereitung bedürfen, können die einzelnen Spannelemente außerhalb des Körpers unter einer vorbestimmten Winkellage entsprechend der Bruchlinie der Fraktur zueinander ausgerichtet und zusammengebaut werden. Hierdurch werden die körperlichen Belastungen für den Patienten erheblich verringert.

Werden eine Vielzahl von Spannelementen bereits vor der Operation zu einer Fixierungsvorrichtung zusammengebaut, führt dies zur Zeitersparnis während der im Anschluss folgenden Operation, wodurch die Belastung für den Patienten weiter verringert wird. Es müssen dabei geringere Mengen an Narkosemittel aufgrund der kürzeren Operation dem Patienten verabreicht werden.

Indem die Vorbereitung der Fixierungsvorrichtung bereits vor der Operation durchgeführt wird, schwindet auch das Fehlerrisiko den Patient zu verletzen oder die Fixierungsvorrichtung fehlerhaft zu erstellen. Die im Voraus erstellte Fixierungsvorrichtung kann zum einen durch mehrere Qualitätsprüfungen durchlaufen, welche von einem dafür geschultes Fachpersonal durchgeführt werden. Eine aufwendige Qualitätsprüfung ist hingegen während einer Operation wegen der begrenzten Zeit und des begrenzt zur Verfügung stehenden Platzes nur sehr schwierig möglich. Zum anderen können die Spannelemente in Ruhe ohne Zeitdruck von den Ärzten zusammengebaut werden, was das Fehlerrisiko gleichfalls mindert.

Obgleich vorstehend zwei Befestigungsmittel verwendet werden, können ferner drei Befestigungsmittel zur Verbesserung der Fixierung der Fixiervorrichtung am Knochen verwendet werden.

Neben der Positionierung der Spannelemente unter einem vorbestimmten Winkel ist die Fixierungsvorrichtung darüber hinaus in der Lage, die wenigstens zwei benachbarten Spannelemente bezüglich deren Längsachsen auszurichten.

Weitere Ausgestaltungen der erfindungsgemäßen Fixierungsvorrichtung sind Gegenstand der abhängigen Ansprüche 2 bis 22.

Ferner kann die Winkelarretierhülse formschlüssig mit den wenigstens zwei Spannelementen verbunden sein. Anhand des durch die Winkelarretierhülse und den wenigstens zwei Spannelementen hergestellten Formschlusses kann besonders gut eine Arretierung vorgesehen werden.

Der Formschluss kann dabei so durchgeführt werden, dass an einer Innenfläche wenigstens eines stirnseitigen Aufnahmeabschnitts des Spannelements wenigstens ein Vorsprung und/oder wenigstens eine Vertiefung ausgebildet ist, und dass an einer Außenfläche der Winkelarretierhülse wenigstens ein Vorsprung und/oder eine Vertiefung ausgebildet ist. Die Vorsprünge bzw. Vertiefungen der Spannelemente sind korrespondierend zu den Vorsprüngen bzw. Vertiefungen der Winkelarretierhülse ausgebildet. Auf diese Weise kann besonders leicht ein Formschluss vorgesehen werden.

Besonders gute Ereignisse zeigten sich jedoch, wenn entlang einer Innenfläche wenigstens eines stirnseitigen Aufnahmeabschnitts des Spannelements wenigstens teilweise eine Innenverzahnung ausgebildet ist, und wenn entlang einer Außenfläche der Winkelarretierhülse wenigstens teilweise eine Außenverzahnung ausgebildet ist. Die Innenverzahnung der Spannelemente ist korrespondierend zu der Außenverzahnung der Winkelarretierhülse ausgebildet. Je feiner die Teilungen der jeweiligen Verzahnungen gewählt werden, desto kleinere Winkelschritte bei der Arretierung wenigstens zweier benachbarter Spannelemente sind möglich.

Darüber hinaus kann wenigstens ein stirnseitiger Aufnahmeabschnitt des Spannelements verjüngt, insbesondere konisch, ausgebildet sein. Mit dieser Ausbildung kann beim Einbringen eines Befestigungsmittels, insbesondere einer Corticalisschraube mit einem zu den Aufnahmeabschnitt korrespondierenden Schraubenkopf, eine Winkelstabilisierung betreffend dem Winkel zwischen Befestigungsmittel und Spannelement vorteilhaft erreicht werden. Die verjüngte Ausbildung des stirnseitigen Aufnahmeabschnitts umfasst auch deren Vorsprünge bzw. Vertiefungen respektive Innenverzahnung.

Zudem können die wenigstens zwei Spannelemente wenigstens mittels der stirnseitigen Aufnahmeabschnitte nach einer Art Nut- und Federverbindung aneinanderkoppelbar sein. Die Spannelemente können so zuverlässig und auf einfache Weise miteinander gekoppelt werden. Anhand dieser Konfiguration kann besonders gut ein Spannelement-Modularsystem bereitgestellt werden.

Überdies kann die Winkelarretierhülse kraftschlüssig mit den wenigstens zwei Spannelementen verbunden sein. Die aufeinander wirkenden Flächen der Winkelarretierhülse und des Spannelements können so konditioniert sein, dass sie besonders gut einen Reibschluss ermöglichen. Daneben kann die Winkelarretierhülse auch so ausgebildet sein, dass sie derart stark gegen die benachbarten Spannelemente drückt, dass die Spannelemente aufgrund der Reibung bezüglich ihrer Winkellage fest zueinander arretiert sind. Die Winkelarretierhülse kann dabei einen größeren äußeren Umfang als der fluchtende Bereich der Spannelemente aufweisen.

Um die Winkelstabilisierung zwischen mehreren Befestigungsmitteln, insbesondere Corticalisschrauben, und einem Spannelement noch weiter zu verbessern, kann an einer Innenfläche der Winkelarretierhülse ein Gewinde ausgebildet sein. Hierdurch stellen die Winkelarretierhülse und die Spannelemente eine noch bessere Wirkverbindung betreffend der Winkelstabilisierung dar.

Eine weitere Möglichkeit ist, die Winkelarretierhülse mit einem derartigen Material auszubilden, dass in deren Innenfläche vermittels eines Befestigungsmittels ein Gewinde eindrehbar ist. Das Befestigungsmittel weist dabei die korrespondierende Struktur der Innenfläche der Winkelarretierhülse auf. Somit kann bei einem Einbringen des Befestigungsmittels in die Bruchenden des Knochens gleichzeitig ein Gewinde in die Innenfläche der Winkelarretierhülse geschnitten werden. Die Winkelstabilität ist hierbei im Vergleich zu einem bereits vorab in die Innenfläche der Winkelarretierhülse geschnittenen Gewinde deutlich höher, da kein Spiel bzw. Freiräume zwischen den beiden Komponenten vorhanden sind.

Ferner kann die Innenfläche und/oder die Außenfläche bzw. Außenverzahnung der Winkelarretierhülse wenigstens teilweise, insbesondere konisch, verjüngt ausgebildet sein. Auch diese Maßnahme verbessert die Winkelstabilität zwischen Knochenplatten und Befestigungsmittel. Bei dieser verjüngten Ausbildung der Innenfläche kann auch verstanden werden, dass das in die Innenfläche geschnittene Gewinde verjüngt ausgebildet ist, wodurch ein konisches Gewinde vorliegt. Obgleich vorstehend von Außenverzahnung gesprochen wird, ist es ohne Weiteres denkbar, dass ein einzelner Vorsprung oder eine Vielzahl an Vorsprüngen verjüngt ausgebildet sind.

Um die Winkelarretierhülse auf einfache Weise in einen durch wenigstens zwei benachbarten Spannelemente hergestellten fluchtenden Bereich einzusetzen, kann die Winkelarretierhülse entlang deren Längsachse einen durchgängigen Schlitz aufweisen. Die Winkelarretierhülse ist somit bezüglich ihres Umfangs zusammendrückbar, wodurch sich ihr Umfang wenigstens soweit verkleinert, dass sie besonders leicht in den fluchtenden Bereich der wenigstens zwei benachbarten Spannelemente eingesetzt werden kann. Obgleich hier von Einsetzen der Winkelarretierhülse in den fluchtenden Bereich gesprochen wird, ist es selbstverständlich möglich, dass die einzelnen Spannelemente auch auf die Winkelarretierhülse angeordnet werden können. Beim Einbringen eines Befestigungsmittels, das einen größeren Umfang als ein innerer Umfang der Winkelarretierhülse aufweist bewirkt der durchgängige Schlitz darüber hinaus, dass sich die Winkelarretierhülse nach außen verformt lässt. Auf diese Weise kann eine Spreizung der Hülse erzeugt werden, die auf die Spannelemente wirkt, und somit die feste Ausrichtung der benachbarten Spannelemente unter einem vorbestimmten Winkel weiter erhöht werden.

Zur Verbesserung der Spreizwirkung der Winkelarretierhülse beim Fixieren an den Bruchenden des Knochens kann die Winkelarretierhülse entlang deren Längsachse wenigstens einen nicht-durchgängigen Schlitz aufweisen. Der durchgängige Schlitz sowie der wenigstens eine nicht-durchgängige Schlitz können in Umfangsrichtung in einem vorbestimmten oder nicht-vorbestimmten Abstand angeordnet sein.

Alternativ kann die Winkelarretierhülse quer zu deren Längsachse einen Manteleinstich aufweisen. Dieser Manteleinstich nimmt zum einen die zwischen den verbundenen Spannelementen auftretenden Biegespannungen und zum anderen die aus der Verformung der Winkelarretierhülse resultierenden Spannungen auf. Auf vorteilhafte Weise kann demzufolge ein Spannungsabbau innerhalb der Fixierungsvorrichtung ermöglicht werden.

Überdies kann die Winkelarretierhülse wenigstens eine wenigstens teilweise umlaufende Kröpfung aufweisen. Wird die Winkelarretierhülse mit dem die Kröpfung ausgebildeten Ende auf die Oberfläche des Knochens positioniert, kann sie die Spannelemente aufnehmen und vom Knochen beabstanden. Die Beabstandung der Spannelemente vom Knochen vermeidet somit, dass die dünnwandige Wandung der Spannelemente die Knochenoberfläche verletzt. Ferner ermöglicht die ausgebildete Kröpfung, dass beim Einsetzen der Winkelarretierhülse in den fluchtenden Bereich der Spannelemente die Winkelarretierhülse nicht durchrutscht.

Die Winkelarretierhülse kann zudem mit einer Lasche ausgebildet sein, vermittels der ein Sicherungsring die Winkelarretierhülse gegenüber wenigstens einem Spannelement sichert. Mittels der an einem Ende der Winkelarretierhülse ausgebildeten Kröpfung und der am anderen Ende der Winkelarretierhülse ausgebildeten Lasche zusammen mit dem Sicherungsring können die benachbarten Spannelemente in deren Höhenrichtung fest miteinander verbunden werden.

Darüber hinaus kann die Winkelarretierhülse verformbar sein, so dass diese beim Einsetzen der Winkelarretierhülse zunächst zusammen gedrückt werden kann und somit das Einsetzen erleichtert wird. Sie kann jedoch auch nach oder während des Einbringens des Befestigungsmittels gespreizt werden und somit die feste Ausrichtung der Winkellage zwischen den Spannelementen noch weiter verstärkt werden. Die Verformung kann dabei sowohl elastisch als auch plastisch sein, wobei die plastische Verformung erst nach Einbringen des Befestigungsmittels erwünscht ist.

Ferner kann die Winkelarretierhülse radial in zwei Abschnitte aufgeteilt sein, und ein radial innerer Abschnitt oder ein radial äußerer Abschnitt aus einem resorbierbaren Kunststoff ausgebildet sein. Der resorbierbare Kunststoff ist ein Material, das von dem umgebenden Körpergewebe abgebaut wird. Resorbierbare Kunststoffe weisen jedoch relativ spröde Materialeigenschaften auf. Sie sind daher nur bedingt zur Ausbildung von Bereichen geeignet, die mechanischen Belastungen ausgesetzt sind. Zudem sind derartige Materialien auch nicht zur Ausbildung elastischer Körper vorgesehen. Durch die Resorption einer der beiden Abschnitte entsteht ein Spiel zwischen dem Befestigungsmittel und dem Spannelement, das zu einer Kraftentkopplung zwischen den Befestigungsmitteln führt. Wenn vorzugsweise ein äußerer Abschnitt aus einem Metall und ein innerer Abschnitt aus einem resorbierbaren Kunststoff besteht, verbleibt mittels der Kröpfungen des äußeren Abschnitts noch eine lokale Eingrenzung des Spannelements. Hierdurch kann ein Lösen des Spannelements aus seiner Anordnung bzw. eine potenzielle Beeinträchtigen des angrenzenden Köpergewebes verhindert werden.

Darüber hinaus kann durch die Winkelarretierhülse ein Befestigungsmittel hindurchgesteckt werden. Die Spannelemente können demgemäß nicht nur miteinander verbunden und zueinander in einer vorbestimmten Winkellage ausgerichtet werden, sondern auch im fluchtenden Bereich mittels eines Befestigungsmittels dort im Knochen eingebracht und befestigt werden. Das Befestigungsmittel kann dabei eine herkömmliche Corticalisschraube sein.

Außerdem können die Corticalisschrauben einen mit einem Gewinde versehenen Schraubenkopf aufweisen. Demnach wird eine Winkelstabilisierung zwischen Corticalisschrauben, insbesondere im Bereich ihres Schraubenkopfes, und des wenigstens einen Spannelements erreicht. Der Schraubenkopf kann dabei als Kreiskeilprofil, Kreuz, Schlitz, Torx, Inbus oder dergleichen ausgebildet sein.

Besonders gut hat sich hinsichtlich der angesprochenen Winkelstabilisierung erwiesen, dass der Schraubenkopf der Corticalisschrauben im Vergleich zu deren Schraubenschaft eine andere Gewindesteigung aufweist. Ist bei einer Corticalisschraube der Schraubenkopf mit einer feineren Gewindesteigung als der Schaft ausgebildet, kann die Wirkverbindung zwischen Corticalisschraube, insbesondere des Schraubenkopfes, und der Winkelarretierhülse aufgrund von zusätzlichen Verschränkungen zueinander und/oder mechanischen Deformationen der Winkelarretierhülse verbessert werden.

Eine weitere Verbesserung der Winkelstabilität ist darin zu sehen, dass der Schraubenkopf der Corticalisschraube verjüngt, insbesondere konisch, ausgebildet ist. Durch diese kreiskegelförmige Ausgestaltung des Schraubenkopfes werden die von der Corticalisschraube zu übertragenden Kräfte besonders gut auf das Spannelement übertragen.

Die vorstehend beschriebenen Merkmale und Funktionen der vorliegenden Erfindung sowie weitere Aspekte und Merkmale werden nachfolgend anhand einer detaillierten Beschreibung von bevorzugten Ausführungsformen unter Bezugnahme auf die beigefügten Figuren weiter beschrieben. Hierbei zeigt:
- Fig. 1: eine perspektivische Ansicht einer ersten Ausführungsform eines erfindungsgemäßen Spannelements;
- Fig. 2A: eine Draufsicht der ersten Ausführungsform des erfindungsgemäßen Spannelements nach Fig. 1;
- Fig. 2B: eine Seitenansicht der ersten Ausführungsform des erfindungsgemäßen Spannelements nach Fig. 1;
- Fig. 2C: eine Seitenansicht einer zweiten Ausführungsform eines alternativen Spannelements;
- Fig. 2D: eine Seitenansicht einer dritten Ausführungsform eines alternativen Spannelements;
- Fig. 3A: eine Draufsicht einer vierten Ausführungsform eines erfindungsgemäßen Spannelements;
- Fig. 3B: eine Schnittansicht des Schnitts B-B der vierten Ausführungsform des erfindungsgemäßen Spannelements gemäß Fig. 3A;
- Fig. 3C: eine Schnittansicht des Schnitts A-A der vierten Ausführungsform des erfindungsgemäßen Spannelements gemäß Fig. 3A;
- Fig. 4A: eine perspektivische Ansicht einer ersten Ausführungsform einer erfindungsgemäßen Winkelarretierhülse;
- Fig. 4B: eine Schnittansicht der ersten Ausführungsform der erfindungsgemäßen Winkelarretierhülse gemäß Fig. 4A;
- Fig. 5A: eine perspektivische Ansicht einer zweiten Ausführungsform einer erfindungsgemäßen Winkelarretierhülse;
- Fig. 5B: eine Schnittansicht der zweiten Ausführungsform der erfindungsgemäßen Winkelarretierhülse gemäß Fig. 5A;
- Fig. 5C: eine Draufsicht der zweiten Ausführungsform der erfindungsgemäßen Winkelarretierhülse gemäß Fig. 5A;
- Fig. 5D: eine perspektivische Ansicht eines Sicherungsrings;
- Fig. 5E: eine Schnittansicht des Sicherungsring gemäß Fig. 5D;
- Fig. 6A: eine perspektivische Ansicht einer dritten Ausführungsform einer erfindungsgemäßen Winkelarretierhülse;
- Fig. 6B: eine Draufsicht der dritten Ausführungsform der erfindungsgemäßen Winkelarretierhülse gemäß Fig. 6A;
- Fig. 7: eine Seitenansicht einer Corticalisschraube;
- Fig. 8A: eine perspektivische Ansicht einer Ausführungsform einer erfindungsgemäßen Fixierungsvorrichtung; und
- Fig. 8B: eine Draufsicht der Ausführungsform der erfindungsgemäßen Fixierungsvorrichtung gemäß Fig. 8A.

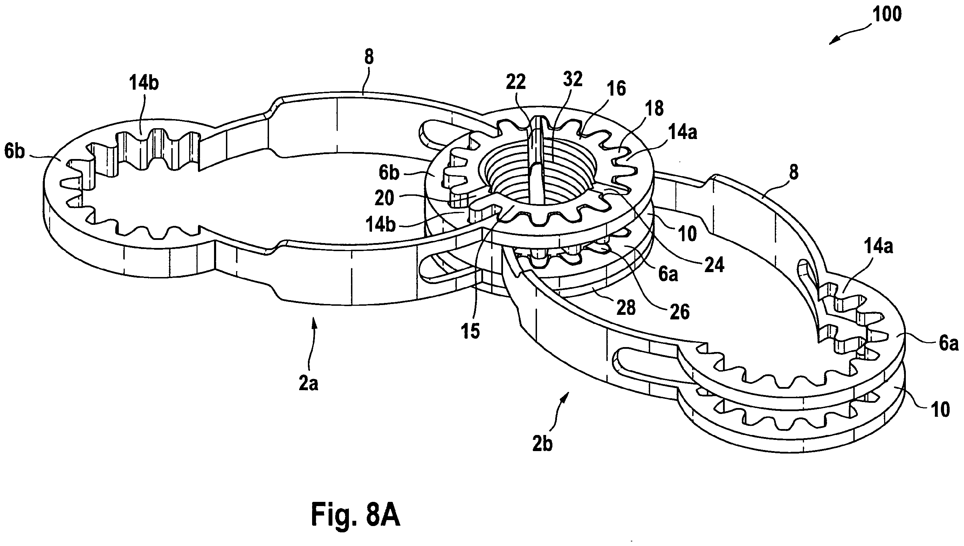

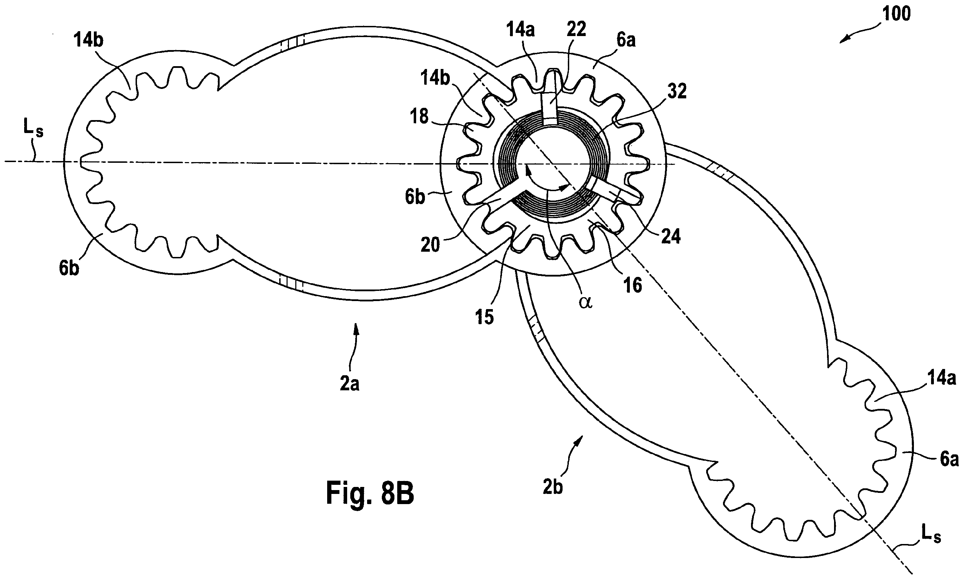

Bevor die Wirkungsweise der Fixierungsvorrichtung 100 in den Fig. 8A und 8B erläutert wird, wird zunächst auf die einzelnen Komponenten eingegangen.

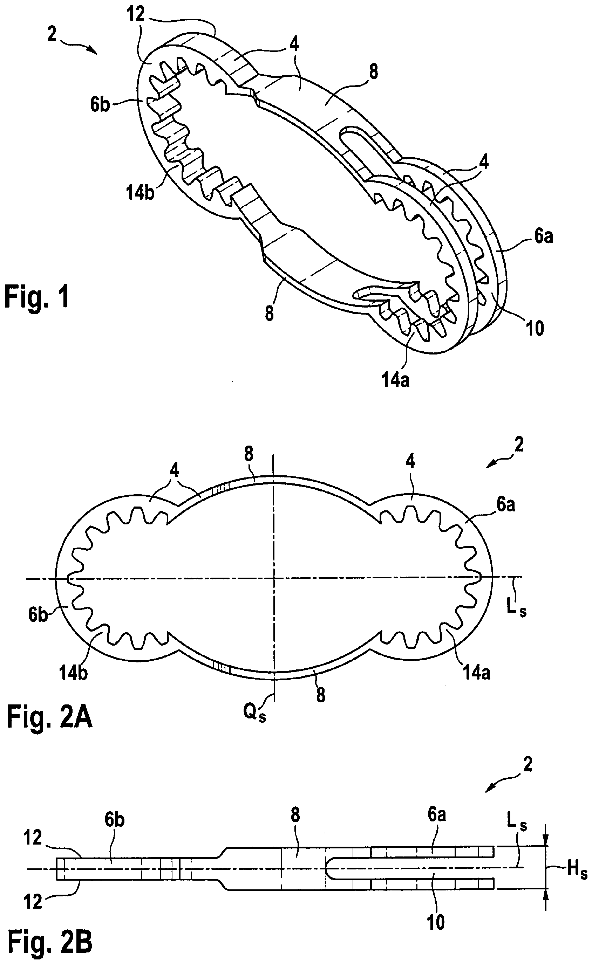

Fig. 1 stellt eine perspektivische Ansicht und Fig. 2A stellt eine Draufsicht einer ersten Ausführungsform eines erfindungsgemäßen Spannelements 2 dar. Das Spannelement 2 besteht aus einem in Draufsicht hohlen, eine umlaufende Wandung 4 aufweisenden Konturkörper. Das Spannelement 2 weist ferner zwei einander gegenüberliegende stirnseitige Aufnahmeabschnitte 6a, 6b und zwei Seitenflanken mit Beugeabschnitten 8 auf. Die zwei stirnseitigen Aufnahmeabschnitte 6a, 6b liegen bezüglich der Querachse Qₛ des Spannelements 2 einander gegenüber und weisen jeweils eine nach außen weisende Krümmung auf. Die zwei Beugeabschnitte 8 liegen bezüglich der Längsachse Lₛ des Spannelements 2 einander gegenüber und weisen jeweils eine nach außen weisende Krümmung auf. Das in Fig. 1 gezeigte Spannelement 2 ist im Wesentlichen rautenförmig ausgebildet.

Die Wandung 4 des Spannelements 2 weist im Bereich des stirnförmigen Aufnahmeabschnitts 6a sowie teilweise in den Seitenflanken einen Durchbruch 10 und im Bereich des stirnförmigen Aufnahmeabschnitts 6b sowie teilweise in den Seitenflanken Ausschnitte 12 auf. Deutlich zu erkennen ist diese Ausgestaltung in der Seitenansicht der Fig. 2B. Der Durchbruch 10 und die Ausschnitte 12 sind derart korrespondierend zueinander ausgebildet, dass durch diese Nut-Gestalt und/oder eine Feder-Gestalt von Wandungsabschnitten eine komplementäre Passung bereitgestellt wird, vermittels der sich die Konturen der benachbarten Spannelemente 2 in Überschneidung bringen lassen. In Fig. 2B ist darüber hinaus die Höhe Hₛ des Spannelements 2 dargestellt. Obwohl das Spannelement 2 Ausschnitte 12 und einen Durchbruch 10 aufweist, ist unter der in Fig. 1, 2A und 2B gezeigten Wandung eine umlaufende Wandung 8 zu verstehen.

Ferner können der Durchbruch 10 und die Ausschnitte 12 anders als bei der ersten Ausführungsform lediglich im Bereich der Aufnahmeabschnitte 6a, 6b ausgebildet sein.

Die stirnseitigen Aufnahmeabschnitte 6a, 6b weisen jeweils eine Innenverzahnung 14a, 14b auf. Der stirnseitige Aufnahmeabschnitt 6a und dessen Innenverzahnung 14a werden mittels des Durchbruchs 10 in zwei Teile geteilt, wobei die einzelnen Zähne der Innenverzahnung 14a bei Betrachtung der Draufsicht von Fig. 2a genau übereinander liegen.

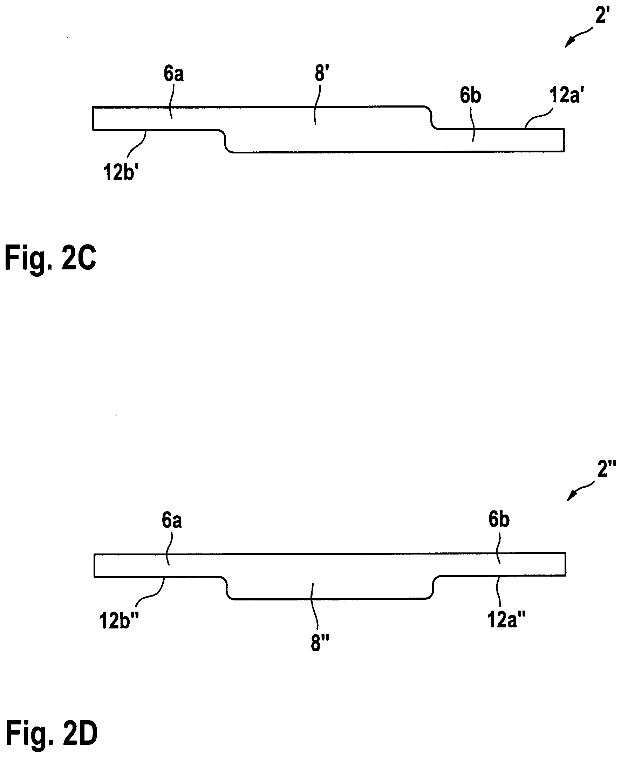

Alternativ zu dem Durchbruch 10 und den Ausschnitten 12 der ersten Ausführungsform des Spannelements, werden in Fig. 2C und 2D eine zweite und eine dritte Ausführungsform des Spannelements 2', 2" aufgezeigt. Die Ausschnitte 12a' 12b' des alternativen Spannelements 2' aus Fig. 2C liegen bei Betrachtung der Seitenansicht diametral in den jeweiligen Aufnahmeabschnitten 6a, 6b gegenüber. Die Ausschnitte 12a" und 12b" des alternativen Spannelements 2" aus Fig. 2D liegen bei Betrachtung der Seitenansicht in den jeweiligen Aufnahmeabschnitten 6a, 6b spiegelbildlich zueinander gegenüber. Die in Fig. 2C und 2D gezeigten Ausgestaltungen der stirnseitigen Aufnahmeabschnitte 6a, 6b der Spannelements 2', 2" ermöglicht gleichfalls das Aneinanderkoppeln von mehreren Spannelementen 2', 2" zu einer Fixierungsvorrichtung 100.

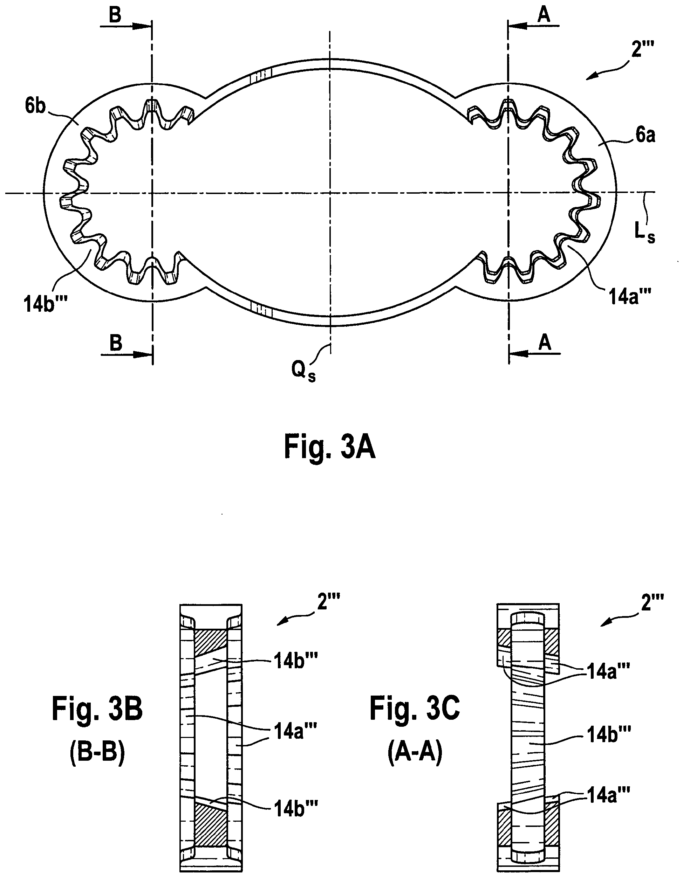

Fig. 3A stellt eine Draufsicht einer vierten Ausführungsform eines erfindungsgemäßen Spannelements 2''' dar, wobei Fig. 3B und 3C die Schnitte B-B und A-A aus Fig. 3A darstellen. Das Spannelement 2''' ist ähnlich wie das Spannelement 2 aus Fig. 1, 2A und 2B ausgebildet. Der einzige Unterschied besteht darin, dass Innenverzahnungen 14a''', 14b''' konisch verjüngt ausgebildet sind, wie durch die Fig. 3B und 3C ersichtlich ist. Die Anordnung des mit einer verjüngten Innenverzahnung 14a"', 14b''' ausgebildeten Spannelements 2''' erfolgt so, dass der verjüngte Bereich der Knochenoberfläche zugewandt ist, um beim Einbringen der Befestigungsmittel eine verbesserte Winkelstabilität zu erreichen.

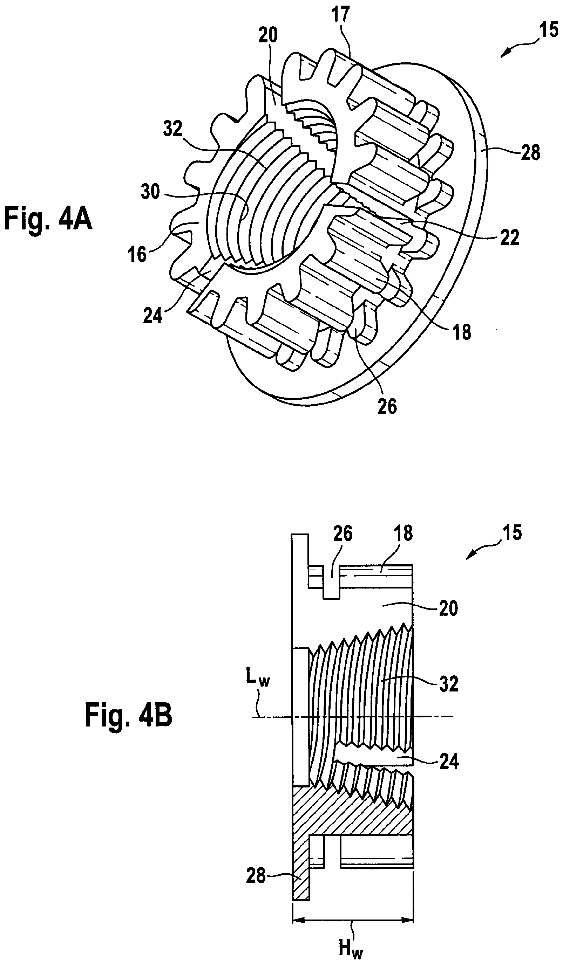

Fig. 4A stellt eine perspektivische Ansicht und Fig. 4B stellt eine Schnittansicht einer ersten Ausführungsform einer erfindungsgemäßen Winkelarretierhülse 15 dar. Die Winkelarretierhülse 15 weist an deren Außenfläche 17 eine umlaufende Außenverzahnung 18 auf, die sich über die Höhe Hw der Winkelarretierhülse 15 erstreckt. Eine Wandung 16 der Winkelarretierhülse 15 ist somit integral mit der Außenverzahnung 18 ausgebildet. Die Wandung 16 und die Außenverzahnung 18 werden von einem durchgängigen Schlitz 20 und zwei nicht-durchgängigen Schlitze 22, 24 unterbrochen. Sowohl der durchgängige Schlitz 20 als auch die nicht-durchgängigen Schlitze 22, 24 verlaufen in Richtung der Längsachse Lw der Winkelarretierhülse 15.

Die Außenverzahnung 18 ist ferner durch einen Manteleinstich 26 unterbrochen, der quer zur Längsachse L_{w} der Winkelarretierhülse 15 verläuft. Der Manteleinstich 26 ist vorgesehen, um zum einen die zwischen zwei benachbarten Spannelementen auftretenden Biegespannungen aufzunehmen. Zum anderen kann sie auch die durch die Spreizung der Winkelarretierhülse 15 hervorgerufenen Spannungen aufnehmen. Aus Fig. 4B ist zu erkennen, dass der Manteleinstich 26 nicht mittig bezüglich der Höhe Hw der Winkelarretierhülse 15 ausgebildet ist. So können die Biegespannungen noch wirksamer aufgenommen werden.

Bei der Winkelarretierhülse 15 ist an einem Ende bezüglich der Längsachse Lw der Winkelarretierhülse 15 eine umlaufende Kröpfung 28 ausgebildet. Die Winkelarretierhülse 15 kann in einem fluchtenden Bereich von mehreren Spannelementen so eingesetzt werden, dass die Kröpfung 28 dem Knochen zugewandt ist. Somit bildet die Kröpfung 28 eine Auflage für die Spannelemente 2 und schont den Knochen dadurch. Darüber hinaus kann die Kröpfung 28 dazu dienen, dass beim Einsetzen der Winkelarretierhülse 15 in den fluchtenden Bereich von mehreren benachbarten Spannelementen 2 die Winkelarretierhülse 15 nicht durchrutscht.

Obschon der Manteleinstich 26 bezogen auf die Höhe Hw der Winkelarretierhülse 15 näher an dem Ende mit der Kröpfung 28 ausgebildet ist, kann er auch ohne Weiteres mittig oder im Bereich des der Kröpfung 28 abgewandten Endes ausgebildet sein.

An einer Innenfläche 30 der Winkelarretierhülse 15 ist ein Gewinde 32 ausgebildet. Besagtes Gewinde 32 verjüngt sich konisch in Richtung der Kröpfung 28 und dient zur Winkelstabilisierung zwischen einem eingeschraubten Befestigungsmittel und dem Spannelement.

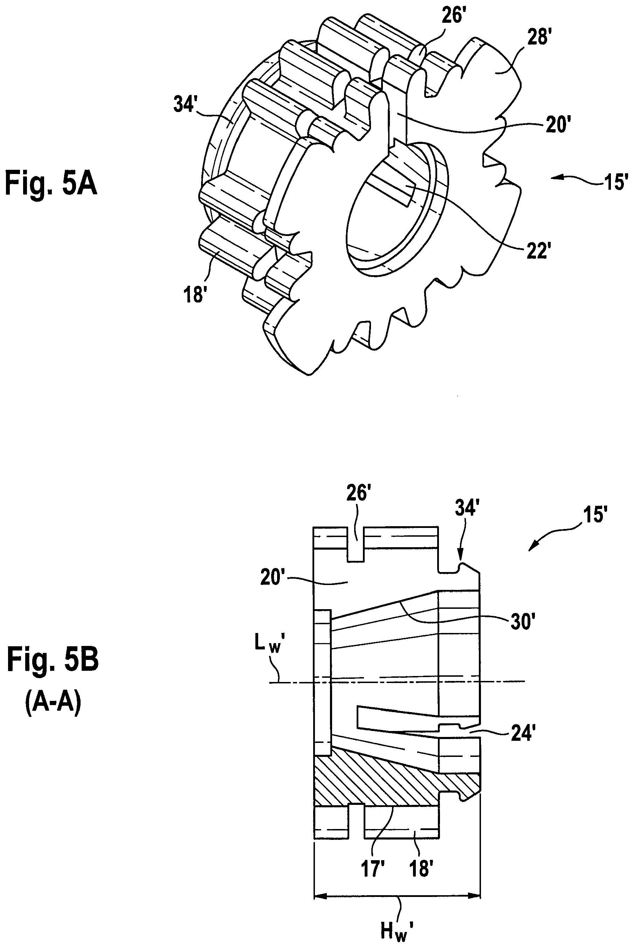

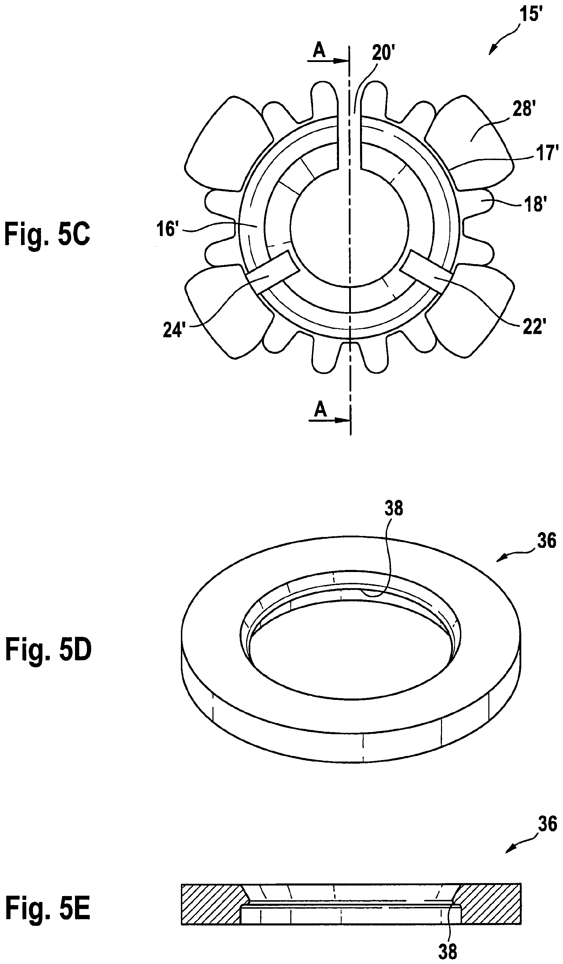

Fig. 5A stellt eine perspektivische Ansicht, Fig. 5B stellt eine Schnittansicht und Fig. 5C stellt eine Draufsicht einer zweiten Ausführungsform einer erfindungsgemäßen Winkelarretierhülse 15' dar. Die Winkelarretierhülse 15' weist an deren Außenfläche 17' eine teilweise umlaufende Außenverzahnung 18' auf. Eine Wandung 16' der Winkelarretierhülse 15' ist somit integral mit der Außenverzahnung 18' ausgebildet. Die Wandung 16' und teilweise die Außenverzahnung 18' werden von einem durchgängigen Schlitz 20' und zwei nicht-durchgängigen Schlitze 22', 24' unterbrochen. Die Schlitze 20', 22' 24' verlaufen in Richtung der Längsache L_{w} der Winkelarretierhülse 15'.

Eine teilweise umlaufende Kröpfung 28' ist an einem freien Ende der Winkelarretierhülse 16' ausgebildet. In der Nähe der teilweise umlaufenden Kröpfung 28' ist quer zur Längsachse L_{w}' der Winkelarretierhülse 15' ein Manteleinstich 26' zur Aufnahme von Biegespannungen ausgebildet.

Bei dem mit der Kröpfung 28' ausgebildeten abgewandten Ende ist eine umlaufende Lasche 34' ausgebildet. Diese dient zur Sicherung unter Zuhilfenahme eines nachstehend beschriebenen Sicherungsrings 36 der Winkelarretierhülse 15' gegenüber den Spannelementen 2.

Eine ein Befestigungsmittel aufweisende Innenfläche 30' der Winkelarretierhülse 15' ist glatt und teilweise konisch verjüngend ausgebildet.

Die Winkelarretierhülse 15' wird auf die Knochenoberfläche so positioniert, dass die umlaufende Kröpfung 28' der Oberfläche des Knochens zugewandt ist. Die Kröpfung 28' nimmt dabei mehrere Spannelemente auf und schützt die empfindliche Oberfläche des Knochens gegenüber der dünnen stirnseitigen Wandung 4 der Spannelemente. Nach dem Aufbringen der Spannelemente auf die Winkelarretierhülse 15' wird der Sicherungsring 36 unter Zuhilfenahme des Vorsprungs 38 mit der Lasche 34' befestigt.

Fig. 5D stellt eine perspektivische Ansicht und Fig. 5E stellt eine Schnittansicht des Sicherungsrings 36 dar. Der Sicherungsring 36 weist einen äußeren Umfang und einem inneren Umfang auf. Im Bereich der Innenfläche des Sicherungsrings 36 ist ein zu der Lasche 34' korrespondierender Vorsprung ausgebildet, um die Sicherung zu ermöglichen.

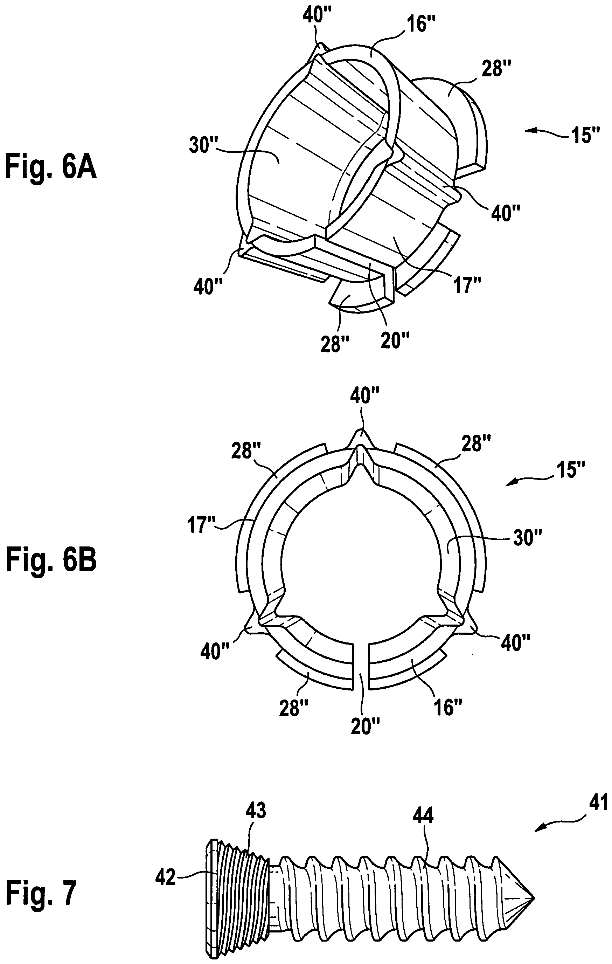

Fig. 6A stellt eine perspektivische Ansicht und Fig. 6B stellt eine Draufansicht einer dritten Ausführungsform einer erfindungsgemäßen Winkelarretierhülse 15" dar. Die Winkelarretierhülse 15" weist eine Wandung 16" auf, die an deren Außenfläche 17" drei in einem vorgegebenen Abstand bezüglich des Umfangs ausgebildete Vorsprünge 40 aufweist. Die Vorsprünge 40" sind in Umfangsrichtung unter einem Winkel von 120° zueinander beabstandet und erstrecken sich über die gesamte Höhe Hw der Winkelarretierhülse 15". Die Winkelarretierhülse 15" weist darüber hinaus einen durchgängigen Schlitz 20" auf. Die Winkelarretierhülse 15" ist über deren gesamten Höhe Hw konisch verjüngt ausgebildet. An dem verjüngten Ende der Winkelarretierhülse 15" ist eine teilweise umlaufende Kröpfung 28" ausgebildet. Die umlaufende Kröpfung 28" ist in einem Beriech unterbrochen, in dem sich die Vorsprünge 40" über die gesamte Höhe H_{w} der Winkelarretierhülse 15" erstrecken.

Obgleich bei dieser Ausführungsform der Winkelarretierhülse 15" weder ein Manteleinstich noch eine Lasche vorgesehen sind, bedeutet dies jedoch nicht, dass die Winkelarretierhülse 15" nicht dahingehend ausgebildet werden kann.

Beispielhaft wird in Fig. 7 eine Seitenansicht einer Corticalisschraube 41 beschrieben. Die Corticalisschraube 41 weist einen Schraubenkopf 42 mit einem Außengewinde 43 und einen Schraubenschaft 44 ebenfalls mit einem Außengewinde auf. Dabei ist das Außengewinde der Schraubenkopf 42 feiner ausgebildet als das Außengewinde des Schraubenschafts 44. Der Schraubenkopf ist in Richtung des Schraubenschafts 44 verjüngt.

Fig. 8A stellt eine perspektivische Ansicht und Fig. 8B stellt eine Draufsicht einer Ausführungsform einer erfindungsgemäßen Fixierungsvorrichtung 100 dar. Die Fixierungsvorrichtung 100 besteht aus zwei Spannelementen 2 nach deren ersten Ausführungsform und einer Winkelarretierhülse 15 nach deren ersten Ausführungsform. Die zwei Spannelemente bilden durch den stirnseitigen Aufnahmeabschnitt 6a eines ersten Spannelements 2a und den stirnseitigen Aufnahmeabschnitt 6b des zweiten Spannelements 2b einen fluchtenden Bereich. In diesen Bereichen wird die Winkelarretierhülse 15 eingesetzt und arretiert die benachbarten Spannelemente 2a, 2b unter einem vorbestimmten Winkel α zueinander. Nach der Winkelarretierung der Spannelemente können die nicht dargestellten Befestigungsmittel durch die Aufnahmeabschnitte 6a, 6b hindurchgesteckt und in die Bruchenden des Knochens eingebracht werden.

Das Spannelement 2, 2',2" und die Winkelarretierhülse 15, 15', 15" werden aus biokompatiblen Materialen hergestellt. Zudem ist ebenfalls ein Hybridaufbau des Spannelements 2, 2',2"möglich, bei dem die Wandung der Beugeabschnitte 8 aus einem Material mit der gewünschten elastischen Eigenschaft bestehen und weitere Bereiche des Spannelements 2, 2',2", wie z.B. die Aufnahmeabschnitte 6a, 6b, aus einem steifen oder resorbierbaren Material gebildet werden.

Als Ausgangsmaterial für das Spannelement 2, 2',2" kommen beispielsweise Metalle aus der Gruppe: X42CrMo15, X100CrMo17, X2CrNiMnMoNNb21-16-5-3, X20Cr13, X15Cr13, X30Cr13, X46Cr13, X17CrNil6-2, X14CrMoS17, X30CrMoN15-1, X65CrMo 17-3, X55CrMo14, X90CrMoV18, X50CrMoV15, X 38CrMo V15, G-X 20CrMol3, X39CrMo17-1, X40CrMoVN16-2, X105CrMo17, X20CrNiMoS13-1, X5CrNi18- 0, XBCrNiS18-9, X2CrNi19-11, X2CrNi18-9, X10CrNi18-8, X5CrNiMo17-12-2, X2CrNiMo17-12-2, X2CrNiMoN25-7-4, X2CrNiMoN17-13-3, X2CrNiMo17-12-3, X2CrNiMo18-14-3, X2CrNiMo18-15-3; X 2 CrNiMo 18 14 3, X13CrMnMoN18-14-3, X2CrNiMoN22136, X2CrNiMnMoNbN21-9-4-3, X4CrNiMnMo21-9-4, X105CrCoMo18-2, X6CrNiTi18-10, X5CrNiCuNb16-4, X3CrNiCuTiNb12-9, X3CrNiCuTiNb12-9, X7CrNiAl17-7, CoCr20Ni15Mo, G-CoCr29Mo, CoCr20W15Ni, Co-20Cr-15W-10Ni, CoCr28MoNi, CoNi35Cr20Mo10, Ti1, Ti2, Ti3, Ti4, Ti-5Al-2,5Fe, Ti-5Al-2,5Sn, Ti-6Al-4V, Ti-6Al-4V ELI, Ti-3Al-2,5V (Gr9), 99,5Ti, Ti-12Mo-6Zr-2Fe, Ti-13,4Al-29Nb, Ti-13Nb-13Zr, Ti-15Al, Ti-15Mo, Ti- 15Mo-5Zr-3Al, Ti-15Sn, Ti-15Zr-4Nb, Ti-15Zr-4Nb-4Ta, Ti-15Zr-4Nb-4Ta-0,2Pd, Ti-29Nb-13Ta-4,6Zr, Ti-30Nb-10Ta-5Zr, Ti-35,SNb-1,5Ta-7,1Zr, Ti-35Zr-10Nb, Ti-45Nb, Ti-30Nb, Ti-30Ta, Ti-6Mn, Ti-5Zr-3Sn-5Mo-15Nb, Ti-3Al-8V-6Cr-4Zr-4Mo, Ti-6Al-2Nb-1Ta-0,8Mo, Ti-6Al-4Fe, Ti-6Al-4Nb, Ti-6Al-6Nb-1Ta, Ti-6Al-7Nb, Ti-6Al-4Zr-2Sn-2Mo, Ti-8,4Al-15,4Nb, Ti-8Al-7Nb, Ti-8Al-1Mo-1V, Ti-11Mo-6Zr-4Sn, in Betracht.

Ferner kommen Polymere aus der Gruppe: MBS, PMMI, MABS, CA, CTA, CAB, CAP, COC, PCT, PCTA, PCTG, EVA, EVAL, PTFE, ePTFE, PCTFE, PVDF, PVF, ETFE, ECTFE, FEP, PFA, LCP, PMMA, PMP, PHEMA, Polyamid 66, Polyamid 6, Polyamid 11 , Polyamid 2, PAEK, PEEK, PB, PC, PPC, PETP, PBT, MDPE, LDPE, HDPE, UHMWPE, LLDPE, PI, PAI, PEI, PIB, POM, PPO, PPE, PPS, PP, PS, PSU, PESU, PVC, PVC-P, PVC-U, ABS, SAN, TPE-U, TPE-A, TPE-E, PVDC, PVA, SI, PDMS, EPM, EP, UF, MF, PF, PUR, UP, PEBA, PHB, PLA, PLLA, PDLA, PDLLA, PGL, PGLA, PGLLA, PGDLLA, PGL-co-poly TMC, PGL-co-PCL, PDS, PVAL, PCL, Poly-TMC, PUR (linear), NiTi Superelastic, NiTi Shape Memory, in Betracht.

Des Weiteren kommen Keramiken aus der Gruppe: Al₂O₃ (Aluminiumoxid), Y-TZP (Zirkonoxidkeramik), AMC (Alumina Matrix Composite), HA (Hydroxilapatit), TCP (Tricalciumphosphat), Ceravital (Glaskeramik/Bioglas®), FZM/K (Zirkonoxid, teilstabilisiert), TZP-A (Zirkonoxidkeramik), ATZ (Alumina-toughened Zirconia), C799 (Aluminiumoxidkeramik), Schott 8625 (Transponderglas), in Betracht.

Darüber hinaus kommen Kombinationen hiervon in Betracht.

Die Erfindung lässt neben den erläuterten Ausführungsformen weitere Gestaltungsansätze zu.

Obgleich die Innenfläche des stirnseitigen Aufnahmeabschnitts des Spannelements oder die Außenfläche der Winkelarretierhülse mit einer Verzahnung ausgebildet sind, können die Vorsprünge bzw. Vertiefungen auch in einer beliebigen vorspringenden bzw. vertiefenden Art ausgebildet sein, wie z.B. noppenförmig, als Wellenprofil oder dergleichen.

Obschon die Winkelarretierhülse vorstehend einstückig ausgebildet ist, ist es ohne Weiteres denkbar, diese auch mehrteilig, z.B. zweiteilig, dreiteilig, auszubilden.

Zwar ist der quer verlaufende Manteleinstich der Winkelarretierhülse bei den Ausführungsformen so ausgebildet, dass er vollständig um die Winkelarretierhülse läuft, jedoch ist es auch möglich, diesen nur in einem Winkel zwischen 90° und 360° auszubilden. Dass der Manteleinstich quer zur Längsachse der Winkelarretierhülse verlaufen muss ist darüber hinaus nicht zwingend.

Die teilweise umlaufende Kröpfung der Winkelarretierhülse kann auch so ausgebildet sein, dass diese auch biegsam ist und an die Knochenoberfläche angeschmiegt werden kann.

Obwohl der Schraubenkopf der Corticalisschraube eine andere Gewindesteigung als der Schraubenschaft der Corticalisschraube aufweist, können diese Gewindesteigungen auch gleich ausgebildet sein.

Die Verzahnungen können als Zykloidenverzahnung, Wildhaber-Novikov-Verzahnung, Evolventenverzahnung oder dergleichen ausgebildet sein.

Sowohl die vorstehend diskutierten Spannelemente als auch die Winkelarretierhülsen können durch Drehen, Fräsen, Erodieren, Spritzguss, Sintern oder dergleichen hergestellt werden.

Obgleich der Schraubenkopf vorstehend konisch ausgebildet ist, kann er auch kreiskeilartig, schiefkreiskeilartig, exzentrisch usw. ausgebildt sein. So können vorteilhaft Oxidationsvorgänge, Diffussionsvorgänge, usw. zwischen dem Schraubenkopf und der Winkelarretierhülse verrringert werden, da nur eine begrentzte Umfangsfläche des Schraubenkopfs mit der Knochenplatte in Kontakt steht.

Die Erfindung schlägt eine Fixierungsvorrichtung zum Fixieren von Bruchenden von Knochen einer Knochenfraktur vor, die wenigstens zwei Spannelementen, welche jeweils einen in Draufsicht hohlen, eine umlaufende Wandung aufweisenden Konturkörper aufweisen, mit zwei einander gegenüberliegenden stirnseitigen Aufnahmeabschnitten, wenigstens einer Spannhülse und wenigstens zwei Befestigungsmittel, welche in den stirnseitigen Aufnahmeabschnitten anordnenbar sind, aufweist. Die wenigstens zwei Spannelemente sind so zueinander angeordnet, dass jeweils ein Aufnahmeabschnitt der Spannelemente hiervon miteinander fluchten und von der Spannhülse derart durchgriffen sind, dass die beiden Spannelemente miteinander gekoppelt sind. Erfindungsgemäß ist ferner die Spannhülse eine Winkelarretierhülse, die derart mit den wenigstens zwei Spannelementen verbunden ist, dass diese in einer vorbestimmten Winkellage zueinander vorliegen.

## Patentansprüche

1. Fixierungsvorrichtung (100) zum Fixieren von Bruchenden von Knochen einer Knochenfraktur, mit
wenigstens zwei Spannelementen (2, 2', 2", 2a, 2b), welche jeweils einen in Draufsicht hohlen, eine umlaufende Wandung (4) aufweisenden Konturkörper aufweisen, der zwei einander gegenüberliegende stirnseitige Aufnahmeabschnitte (6a, 6b) umfasst,
wenigstens einer Spannhülse, und
wenigstens zwei Befestigungsmitteln, welche in den stirnseitigen Aufnahmeabschnitten (6a, 6b) anordenbar sind,
wobei die wenigstens zwei Spannelemente (2, 2', 2", 2a, 2b) so zueinander angeordnet sind, dass einer der beiden stirnseitigen Aufnahmeabschnitte eines der Spannelemente und einer der beiden stirnseitigen Aufnahmeabschnitte des/eines anderen der Spannelemente miteinander fluchten und von der Spannhülse derart durchgriffen sind, dass die beiden Spannelemente (2, ,2', 2", 2a, 2b) miteinander gekoppelt sind,
**dadurch gekennzeichnet,**
**dass** die Spannhülse eine Winkelarretierhülse (15, 15', 15") ist, und
**dass** die Winkelarretierhülse (15, 15', 15") derart mit den wenigstens zwei Spannelementen (2, 2', 2", 2a, 2b) verbunden ist, dass diese in einer vorbestimmten Winkellage zueinander vorliegen.

2. Fixierungsvorrichtung nach Anspruch 1, **dadurch gekennzeichnet, dass** die Winkelarretierhülse (15, 15', 15") formschlüssig mit den wenigstens zwei Spannelementen (2, 2', 2", 2a, 2b) verbunden ist.

3. Fixierungsvorrichtung nach Anspruch 1 oder 2, **dadurch gekennzeichnet, dass** an einer Innenfläche (30, 30' 30") wenigstens eines stirnseitigen Aufnahmeabschnitts (6a, 6b) des Spannelements (2, 2', 2", 2a, 2b) wenigstens ein Vorsprung und/oder wenigstens eine Vertiefung ausgebildet ist, und dass an einer Außenfläche (17, 17', 17") der Winkelarretierhülse (15, 15', 15") wenigstens ein Vorsprung (40") und/oder eine Vertiefung ausgebildet ist.

4. Fixierungsvorrichtung nach einem der Ansprüche 1 bis 3, **dadurch gekennzeichnet, dass** entlang einer Innenfläche (30, 30' 30") wenigstens eines stirnseitigen Aufnahmeabschnitts (6a, 6b) des Spannelements (2, 2', 2", 2a, 2b) wenigstens teilweise eine Innenverzahnung (14a, 14b, 14a''', 14b''') ausgebildet ist, und dass entlang einer Außenfläche (17, 17', 17") der Winkelarretierhülse (15, 15', 15") wenigstens teilweise eine Außenverzahnung (18) ausgebildet ist.

5. Fixierungsvorrichtung nach einem der Ansprüche 1 bis 4, **dadurch gekennzeichnet, dass** wenigstens ein stirnseitiger Aufnahmeabschnitt (6a, 6b) des Spannelements (2, 2', 2", 2a, 2b) verjüngt, insbesondere konisch, ausgebildet ist.

6. Fixierungsvorrichtung nach einem der Ansprüche 1 bis 5, **dadurch gekennzeichnet, dass** die wenigstens zwei Spannelemente (2, 2', 2", 2a, 2b) wenigstens mittels der stirnseitigen Aufnahmeabschnitte (6a, 6b) nach einer Art Nut- und Federverbindung aneinanderkoppelbar sind.

7. Fixierungsvorrichtung nach Anspruch 1, **dadurch gekennzeichnet, dass** die Winkelarretierhülse (15, 15', 15") kraftschlüssig mit den wenigstens zwei Spannelementen (2, 2', 2", 2a, 2b) verbunden ist.

8. Fixierungsvorrichtung nach einem der Ansprüche 1 bis 7, **dadurch gekennzeichnet, dass** an einer Innenfläche (30) der Winkelarretierhülse (15, 15', 15") ein Gewinde (32) ausgebildet ist.

9. Fixierungsvorrichtung nach einem der Ansprüche 1 bis 7, **dadurch gekennzeichnet, dass** die Winkelarretierhülse (15, 15', 15") mit einem derartigen Material ausgebildet ist, dass in deren Innenfläche (30) vermittels eines Befestigungsmittels ein Gewinde (32) eindrehbar ist.

10. Fixierungsvorrichtung nach einem der Ansprüche 3 oder 4, **dadurch gekennzeichnet, dass** die Innenfläche (30, 30' 30") und/oder die Außenverzahnung (18) der Winkelarretierhülse (15, 15', 15") wenigstens teilweise, insbesondere konisch, verjüngt ausgebildet sind.

11. Fixierungsvorrichtung nach einem der Ansprüche 1 bis 10, **dadurch gekennzeichnet, dass** die Winkelarretierhülse (15, 15', 15") entlang deren Längsachse (L_{A}) einen durchgängigen Schlitz (20, 20', 20") aufweist.

12. Fixierungsvorrichtung nach einem der Ansprüche 1 bis 11, **dadurch gekennzeichnet, dass** die Winkelarretierhülse (15, 15', 15") entlang deren Längsachse (L_{A}) wenigstens einen nicht-durchgängigen Schlitz (22, 24, 22', 24', 22", 24") aufweist.

13. Fixierungsvorrichtung nach einem der Ansprüche 1 bis 12, **dadurch gekennzeichnet, dass** die Winkelarretierhülse (15, 15', 15") quer zu deren Längsachse (L_{A}) einen Manteleinstich (26, 26', 26") aufweist.

14. Fixierungsvorrichtung nach einem der Ansprüche 1 bis 13, **dadurch gekennzeichnet, dass** die Winkelarretierhülse (15, 15', 15") wenigstens eine wenigstens teilweise umlaufende Kröpfung (28, 28', 28") aufweist.

15. Fixierungsvorrichtung nach einem der Ansprüche 1 bis 14, **dadurch gekennzeichnet, dass** die Winkelarretierhülse (15, 15', 15") eine Lasche (34') aufweist, vermittels der ein Sicherungsring (36) die Winkelarretierhülse (15, 15', 15") gegenüber wenigstens einem Spannelement (2, 2', 2", 2a, 2b) sichert.

16. Fixierungsvorrichtung nach einem der Ansprüche 1 bis 15, **dadurch gekennzeichnet, dass** die Winkelarretierhülse (15, 15', 15") verformbar ist.

17. Fixierungsvorrichtung nach einem der Ansprüche 1 bis 16, **dadurch gekennzeichnet, dass** die Winkelarretierhülse (15, 15', 15") radial in zwei Abschnitte aufgeteilt ist, und ein radial innerer Abschnitt oder ein radial äußerer Abschnitt aus einem resorbierbaren Kunststoff ausgebildet ist.

18. Fixierungsvorrichtung nach einem der Ansprüche 1 bis 17, **dadurch gekennzeichnet, dass** durch die Winkelarretierhülse (15, 15', 15") ein Befestigungsmittel hindurchsteckbar ist.

19. Fixierungsvorrichtung nach Anspruch 18, **dadurch gekennzeichnet, dass** die Befestigungsmittel Corticalisschrauben (41) sind.

20. Fixierungsvorrichtung nach Anspruch 19, **dadurch gekennzeichnet, dass** die Corticalisschrauben (41) einen mit einem Gewinde (43) versehenen Schraubenkopf (42) aufweisen.

21. Fixierungsvorrichtung nach Anspruch 20, **dadurch gekennzeichnet, dass** der Schraubenkopf (42) der Corticalisschrauben (41) im Vergleich zu deren Schraubenschaft (44) eine andere Gewindesteigung aufweist.

22. Fixierungsvorrichtung nach Anspruch 20 oder 21, **dadurch gekennzeichnet, dass** der Schraubenkopf (42) der Corticalisschraube (41) verjüngt, insbesondere konisch, ausgebildet ist.

## Claims

1. A fixing device (100) for fixing the fracture ends of the bones in a bone fracture, with
at least two tensioning elements (2, 2', 2", 2a, 2b), each of which comprising a contoured body which is hollow when viewed from above, has a circumferential wall (4) and includes two face sided receiving sections (6a, 6b) lying opposite to each other,
at least one clamping sleeve, and
at least two fixing means which can be positioned in the face sided receiving sections (6a, 6b),
wherein the at least two tensioning elements (2, 2', 2", 2a, 2b) are positioned in relation to each other in such a way that one of the two face sided receiving sections of one of the tensioning elements and one of the two face sided receiving sections of the/a other tensioning element are aligned with the respective other and are penetrated by the clamping sleeve in such a way that both tensioning elements (2, 2', 2", 2a, 2b) are coupled to each other,
**characterized in**
**that** the clamping sleeve is an angle clamping sleeve (15, 15', 15"), and
**that** the angle clamping sleeve (15, 15', 15") is connected with the at least two tensioning elements (2, 2', 2", 2a, 2b) in such a way that the latter lie in a predetermined angular position in relation to each other.

2. The fixing device according to claim 1, **characterized in that** the angle clamping sleeve (15, 15', 15") is connected to the at least two tensioning elements (2, 2', 2", 2a, 2b) in a form fitting manner.

3. The fixing device according to claim 1 or 2, **characterized in that** at an inner surface (30, 30', 30") of at least one of the face sided receiving sections (6a, 6b) of the tensioning element (2, 2', 2", 2a, 2b) at least one protrusion and/or at least one recess is formed and that at an outer surface (17, 17', 17") of the angle clamping sleeve (15, 15', 15") at least one protrusion (40") and/or at least one recess is formed.

4. The fixing device according to anyone of claims 1 to 3, **characterized in that** along a inner surface (30, 30', 30") of at least one of the face sided receiving sections (6a, 6b) of the tensioning element (2, 2', 2", 2a, 2b) at least in parts internal teeth (14a, 14b, 14a''', 14b''') are formed and that along an outer surface (17, 17', 17") of the angle clamping sleeve (15, 15', 15") at least in parts external teeth (18) are formed.

5. The fixing device according to anyone of claims 1 to 4, **characterized in that** at least one face sided receiving section (6a, 6b) of the tensioning element (2, 2', 2", 2a, 2b) is formed tapered, in particular conical.

6. The fixing device according to anyone of claims 1 to 5, **characterized in that** the at least two tensioning elements (2, 2', 2", 2a, 2b) can be coupled to each other via the face sided receiving sections (6a, 6b) by a tongue and groove connection.

7. The fixing device according to anyone of claims 1 to 6, **characterized in that** the angle clamping sleeve (15, 15', 15") is connected to the at least two tensioning elements (2, 2', 2", 2a, 2b) in a force fitting manner.

8. The fixing device according to anyone of claims 1 to 7, **characterized in that** a thread (32) is formed on an inner surface (30) of the angle clamping sleeve (15, 15', 15").

9. The fixing device according to anyone of claims 1 to 7, **characterized in that** the angle clamping sleeve (15, 15', 15") is formed from a material which allows screwing in a thread in its inner surface (30) by a fixing means.

10. The fixing device according to anyone of claims 3 or 4, **characterized in that** the inner surface (30, 30', 30") and/or the external teeth (18) of the angle clamping sleeve (15, 15', 15") are at least in parts formed tapered, in particular conical.

11. The fixing device according to anyone of claims 1 to 10, **characterized in that** the angle clamping sleeve (15, 15', 15") comprises a continuous slit (20, 20', 20") along its longitudinal axis (L_{A}).

12. The fixing device according to anyone of claims 1 to 11, **characterized in that** the angle clamping sleeve (15, 15', 15") comprises at least one non-continuous slit (22, 24, 22', 24', 22", 24") along its longitudinal axis (L_{A}).

13. The fixing device according to anyone of claims 1 to 12, **characterized in that** the angle clamping sleeve (15, 15', 15") comprises a lateral surface groove (26, 26', 26") extending transversally to its longitudinal axis (L_{A}).

14. The fixing device according to anyone of claims 1 to 13, **characterized in that** the angle clamping sleeve (15, 15', 15") comprises at least one at least in parts circumferential crank (28, 28', 28").

15. The fixing device according to anyone of claims 1 to 14, **characterized in that** the angle clamping sleeve (15, 15', 15") comprises a flap (34') by which a locking ring (36) retains the angle clamping sleeve (15, 15', 15") with respect to at least one tensioning element (2, 2', 2", 2a, 2b).

16. The fixing device according to anyone of claims 1 to 15, **characterized in that** the angle clamping sleeve (15, 15', 15") is deformable.

17. The fixing device according to anyone of claims 1 to 16, **characterized in that** the angle clamping sleeve (15, 15', 15") is divided in radial direction into two sections and the radial inner section or the radial outer section is made of an absorbable synthetic material.

18. The fixing device according to anyone of claims 1 to 17, **characterized in that** a fixing means can be pushed through the angle clamping sleeve (15, 15', 15").

19. The fixing device according to claim 18, **characterized in that** fixing means are cortical screws (41).

20. The fixing device according to claim 19, **characterized in that** the cortical screws (41) comprise a screw head (42) provided with a thread (43).

21. The fixing device according to claim 20, **characterized in that** the screw head (42) of the cortical screws (41) comprises a thread pitch different from the thread pitch of the screw shaft (44).

22. The fixing device according to claim 20 or 21, **characterized in that** the screw head (42) of the cortical screw (41) is formed tapered, in particular conical.

## Revendications

1. Dispositif de fixation (100) pour fixer des extrémités d'os fracturés d'une fracture osseuse, comportant au moins deux éléments de serrage (2, 2', 2", 2a, 2b), qui présentent respectivement un corps de contour creux en vue du dessus, présentant une paroi périphérique (4), qui comprend deux segments de réception (6a, 6b) opposés l'un à l'autre du côté avant,
au mois un manchon de serrage, et
au moins deux moyens de fixation qui peuvent être disposés dans les segments de réception (6a, 6b) du côté avant,
dans lequel les au moins deux éléments de serrage (2, 2', 2", 2a, 2b) sont disposés l'un par rapport à l'autre de telle sorte que l'un des deux segments de réception du côté avant de l'un des éléments de serrage et de l'un des deux segments de réception du côté avant du/de l'un autre élément de serrage sont alignés et sont traversés par le manchon de serrage de telle sorte que les deux éléments de serrage (2, 2', 2", 2a, 2b) sont couplés l'un à l'autre,
**caractérisé en ce que**
le manchon de serrage est un manchon d'arrêt angulaire (15, 15', 15") et **en ce que**
le manchon de serrage angulaire (15, 15', 15") est relié avec les au moins deux éléments de serrage (2, 2', 2", 2a, 2b) de telle sorte que ceux-ci se trouvent l'un par rapport à l'autre dans une position angulaire prédéterminée.

2. Dispositif de fixation selon la revendication 1, **caractérisé en ce que** le manchon d'arrêt angulaire (15, 15', 15") est relié par coopération de forme avec les au moins deux éléments de serrage (2, 2', 2", 2a, 2b).

3. Dispositif de fixation selon la revendication 1 ou 2, **caractérisé en ce que** sur une surface intérieure (30, 30', 30") d'au moins un segment de réception du côté avant (6a, 6b) de l'élément de serrage (2, 2', 2", 2a, 2b), au moins une protubérance et/ou au moins un creux est formé(e), et **en ce que** sur une surface extérieure (17, 17', 17") du manchon d'arrêt angulaire (15, 15', 15"), au moins une protubérance (40") et/ou un creux est formé(e).

4. Dispositif de fixation selon l'une des revendications 1 à 3, **caractérisé en ce que**, le long d'une surface interne (30, 30', 30") d'au moins un segment de réception du côté avant (6a, 6b) de l'élément de serrage (2, 2', 2", 2a, 2b) est formée au moins partiellement une denture intérieure (14a, 14b, 14a''', 14b''') et **en ce que**, le long d'une surface extérieure (17, 17', 17") du manchon d'arrêt angulaire (15, 15', 15") est formée au moins partiellement une denture extérieure (18).

5. Dispositif de fixation selon l'une des revendications 1 à 4, **caractérisé en ce qu'**au moins un segment de réception du côté avant (6a, 6b) de l'élément de serrage (2, 2', 2", 2a, 2b) se rétrécit, en particulier est formé de façon conique.

6. Dispositif de fixation selon l'une des revendications 1 à 5, **caractérisé en ce que** les au moins deux éléments de serrage (2, 2', 2", 2a, 2b) peuvent être couplés l'un à l'autre au moins au moyen des segments de réception du côté avant (6a, 6b) selon une sorte de liaison à rainure et languette.

7. Dispositif de fixation selon la revendication 1, **caractérisé en ce que** le manchon d'arrêt angulaire (15, 15', 15") est relié mécaniquement avec les au moins deux éléments de serrage (2, 2', 2", 2a, 2b).

8. Dispositif de fixation selon l'une des revendications 1 à 7, **caractérisé en ce que** sur une surface intérieure (30) de la gaine d'arrêt angulaire (15, 15', 15") est formé un filetage (32).

9. Dispositif de fixation selon l'une des revendications 1 à 7, **caractérisé en ce que** le manchon d'arrêt angulaire (15, 15', 15") est formé d'un matériau de telle sorte que dans sa surface intérieure (30) un filetage (32) peut être inséré au moyen d'un élément de fixation.

10. Dispositif de fixation selon l'une des revendications 3 ou 4, **caractérisé en ce que** la surface intérieure (30, 30', 30") et/ou la denture extérieure (18) du manchon d'arrêt angulaire (15, 15', 15") sont formées au moins en partie en se rétrécissant, en particulier de façon conique.

11. Dispositif de fixation selon l'une des revendications 1 à 10, **caractérisé en ce que** le manchon d'arrêt angulaire (15, 15', 15") présente le long de son axe longitudinal (L_{A}), une fente continue (20, 20', 20").

12. Dispositif de fixation selon l'une des revendications 1 à 11, **caractérisé en ce que** le manchon d'arrêt angulaire (15, 15', 15") le long de son axe longitudinal (L_{A}) présente au moins une fente non continue (22, 24, 22', 24', 22", 24").

13. Dispositif de fixation selon l'une des revendications 1 à 12, **caractérisé en ce que** le manchon d'arrêt angulaire (15, 15', 15") présente perpendiculairement à son axe longitudinal (L_{A}), une encoche de manteau (26, 26', 26").

14. Dispositif de fixation selon l'une des revendications 1 à 13, **caractérisé en ce que** le manchon d'arrêt angulaire (15, 15', 15") présente au moins une partie coudée partiellement périphérique (28, 28', 28").

15. Dispositif de fixation selon l'une des revendications 1 à 14, **caractérisé en ce que** la gaine d'arrêt angulaire (15, 15', 15") présente une patte (34') au moyen de laquelle un anneau de sécurité (36) sécurise le manchon d'arrêt angulaire (15, 15', 15") contre au moins un élément de serrage (2, 2', 2", 2a, 2b).

16. Dispositif de fixation selon l'une des revendications 1 à 15, **caractérisé en ce que** le manchon d'arrêt angulaire (15, 15', 15") peut être déformé.

17. Dispositif de fixation selon l'une des revendications 1 à 16, **caractérisé en ce que** le manchon d'arrêt angulaire (15, 15', 15") est séparé radialement en deux segments et un segment intérieur sur le plan radial ou un segment extérieur sur le plan radial est formé d'une matière plastique résorbable.

18. Dispositif de fixation selon l'une des revendications 1 à 17, **caractérisé en ce que** le manchon d'arrêt angulaire (15, 15', 15") est un moyen de fixation pouvant être enfiché.

19. Dispositif de fixation selon la revendication 18, **caractérisé en ce que** les moyens de fixation sont des vis corticales (41).

20. Dispositif de fixation selon la revendication 19, **caractérisé en ce que** les vis corticales (41) présentent une tête de vis (42) dotée d'un filetage (43).

21. Dispositif de fixation selon la revendication 20, **caractérisé en ce que** la tête de vis (42) des vis corticales (41) présente par rapport à la tige de vis (44), un autre pas de filetage.

22. Dispositif de fixation selon la revendication 20 ou 21, **caractérisé en ce que** la tête de vis (42) de la vis corticale (41) se rétrécit, en particulier est formée de façon conique.
